Europäisches Patentamt

European Patent Office

Office européen des brevets

(19)

(11) Publication number: **0 260 131**
**A2**

# (12) EUROPEAN PATENT APPLICATION

(21) Application number: **87307987.5**

(22) Date of filing: **09.09.87**

(51) Int. Cl.⁴: **A 61 L 15/00**
**A 41 B 17/00**

(30) Priority: **09.09.86 GB 8621694**

(43) Date of publication of application:
**16.03.88 Bulletin 88/11**

(84) Designated Contracting States:
**AT BE CH DE ES FR GB IT LI LU NL SE**

(71) Applicant: **EXXON CHEMICAL PATENTS INC.**
**1900 East Linden Avenue**
**Linden New Jersey 07036 (US)**

(72) Inventor: **Ohlsson, Stefan Bertil**
**Boulevard Bischoffsheim 39 Bte 13**
**B-1000 Bruxelles (BE)**

**Laus, Guy Guillaume**
**25 Avenue Léopold**
**B-1330 Rixensart (BE)**

(74) Representative: **Dew, Melvyn John et al**
**Exxon Chemical Limited Exxon Chemical Technology**
**Centre P.O. Box 1**
**Abingdon Oxfordshire, OX13 6BB (GB)**

(54) **Garments.**

(57) Disposable liquid absorbent garments such as diapers having elastic grip regions comprise a liquid impervious outer layer, a liquid pervious inner liner and an intermediate absorbent layer. The outer layer is an elastomeric film blend of thermoplastic ethylene polymer and olefinic elastomer which is oriented except for local areas of reduced orientation and increased elasticity which constitute the grip regions.

EP 0 260 131 A2

**Description**

GARMENTS

This invention relates to disposable absorbent garments, in particular for use in the field of hygiene.

Various constructions of liquid-absorbent products such as disposable diapers for babies and adults ("nappies), bed pads, hospital medical and surgical pads and sanitary napkins are known in the art which include a body contacting layer of liquid-pervious materials, an outer liquid-impervious layer and an intermediate liquid-absorbent component which preferably when wetted absorbs many times its own weight of liquid. This absorbent material may be a gel-forming material or a fluffed fibrous pulp or creped, laminated or single ply wadding.

The outer layer has traditionally been formed of a polyolefinic type film and particularly of a polyethylene film (eg EP 0 063 331 or EP 0 082 727), or of a tissue paper impregnated with a water-proofing agent (eg US 3 903 889). GB 2 115 702 uses a vapour permeable, liquid impermeable film comprising polyolefin, filler and liquid or wax-like hydrocarbon polymer or liquid rubber, which film is then stretched. The principal functions of the outer layer is to retain moisture (eg urine or faecal matter) in the absorbent component.

In order to prevent leakages the outer layers have also been designed with inserts such as waist and leg elastomeric strips and bands. The outer layer also dictates how the absorbent product feels when it is in use. Traditional products have not been entirely satisfactory in that they make the wearer uncomfortable. They also have the additional drawback of being noisy when worn.

It is taught in EP-A-0194150, incorporated herein by reference, that an elastomeric film may be used as the outer layer in absorbent products. The elastomeric film may replace polyolefinic film or tissues as the outer layer of such products. This invention also relates to such replacement, but is concerned with garments wherein the elastomeric film has been subjected to orienting processes and localised heat treatment to yield products which have areas of improved grip. Such products are distinguished from conventional ones employing eg a polyolefinic outer film which has been accorded some elasticity or grippiness by adhesion of a strip or band of heat shrinkable elastomeric material thereto.

The present invention is concerned with liquid-absorbent garments of the type which are disposable as, for example, by flushing in a toilet system.

As used herein "absorbent garments" includes diapers for babies and adults, and other pads and medical health or hygiene related devices which come into contact with the body for the purpose of absorbing body fluids or waste and which are required to grip the body or parts thereof in specific places to reduce leakage of such fluids and waste. While various embodiments of the invention are described herein in terms of disposable diaper construction, it will be understood that the invention is applicable in its broadest aspects to other types of liquid-absorbent garments.

In one aspect, the present invention provides a disposable liquid absorbent garment having at least one elastic grip region for improved body fit in use, which garment comprises in combination (a) a liquid impervious outer layer, (b) a liquid permeable liner, and (c) an aborbent layer disposed between the outer layer and the liner, characterised in that the outer layer comprises an elastomeric film comprising a blend of (i) an olefinic elastomer and (ii) a thermoplastic ethylene homopolymer and/or copolymer, and in that the elastomeric film is oriented save for for at least one local area of reduced orientation and increased elasticity which forms the elastic grip region.

In a second aspect, the present invention provides a method of producing a disposable liquid absorbent garment having at least one elastic grip region for improved body fit in use, which method comprises:

(1) providing (i) an elastomeric film comprising a blend of a thermoplastic ethylene homopolymer and/or copolymer and an olefinic elastomer, the film being oriented and being dimensionally stable, thermally unstable in the oriented condition and constractible to a thermally stable and more elastic condition by application of heat, (ii) a liquid permeable sheet, and (iii) an absorbent material;

(2) assembling the film, sheet and absorbent material to form a shaped composite which comprises a liquid impervious outer layer comprising the elastomeric film, a liquid permeable liner comprising the sheet, and an absorbent layer comprising the absorbent material disposed between the outer layer and the liner; and

(3) applying heat to at least one selected local area of the outer layer to reduce the film orientation in that area and thereby contract the film in that area to a thermally stable and more elastic condition to constitute the elastic grip region.

The preferred compositional aspects of the garment per se relate equally to the method, and so will be described herein with reference only to the defined garment although the description will be understood to apply equally to the materials used in the method.

The thermoplastic homopolymer and/or copolymer component of the film blend is preferably a polyethylene, a copolymer of ethylene and an alpha-olefin having 3-16 carbon atoms such as propylene or 1-butene, or an ethylene copolymer formed with an unsaturated comonomer such as an alpha-olefin, a vinyl ester or an olefinically unsaturated acid or ester. The polyethylene may be a low, medium or high density polymer and may be formed by a high pressure, radical process or by a Ziegler catalysed process. Thus, for example, suitable polyethylenes include covnentional low density polyethylene formed by a higher pressure process (LDPE) or the more linear, low density polyethylene prepared by Ziegler catalysed polymerisation (LLDPE). More preferably an ethylene copolymer such as ethylene / vinyl acetate, ethylene/acrylic acid, ethylene / methacrylic acid, ethylene/methyl acrylate or ethylene/butyl acrylate copolymer may be used. Preferably the thermoplastic homopolymer and/or copolymer is employed in the film blend in an amount of 50-90 wt %, more preferably 55-85 wt %, and most preferably 65-80 wt %, based on the weight of thermoplastic and elastomeric components. It is important that the blend contains adequate crystallinity, in order that the film formed from it has the necessary dimensional stability. Thus in general the higher thermoplastics material/lower elastomer material content ranges are to be preferred. Particularly preferred copolymers are ethylene / vinyl acetate copolymers (EVAs) containing from 9 to 40% by weight, especially 15 to 35% of vinyl acetate. In general, blends containing EVA with VA contents below about 9 wt % do not possess sufficient flexibility and orientability for the purposes of the present invention and VA contents above 40 wt % exhibit excessive tackiness. The best balance of orientability and non-tackiness occurs at VA contents between 15 and 35 wt %. Preferably the thermoplastic component, especially the EVAs, have a melt index (ASTM D 1238E) of from 0.1 to 10 g/10 mins, most preferably from 1 to 5 g/10 mins.

The olefinic elastomer component of the film blend is elastomeric and therefore non-liquid and it may be for example an ethylene-alphaolefin copolymer such as an ethylene-propylene elastomer (EPM) (ASTM D-1418-72a designation) or an ethylene- alphaolefin-polyene terpolymer such as an ethylene-propylene-polyene (preferably diene) elastomer (EPDM) (ASTM D-1418-72a designation). The non-conjugated polyene in the terpolymer (EPDM) may be, for example, 1,4-hexadiene, dicyclopentadiene or ethylidene norbornene. The ethylenic elastomers used in the invention preferably have an ethylene content of from 45 to 90 wt %, more preferably 45 to 80 wt % and preferably a Mooney viscosity [ML 1+8 at 127° C] of from 15 to 80.

The invention can employ as the elastomer component various alternative elastomers such as polyisobutylene (PIB), isobutylene-isoprene rubbers (IIR, or "butyl" rubbers) and halogenated derivatives thereof, or thermoplastic rubbers such as styrene-butadiene-styrene block copolymer rubbers (SBS). These may be employed alone or in combination with any of each other or with the copolymers and/or terpolymers mentioned above (EPM and/or EPDM).

Butyl rubber is generally defined as a vulcanizable rubber copolymer containing from 85 to 99.5 per cent combined isoolefin having from 4 to 8 carbon atoms and from 0.5 to 15 per cent combined conjugated diolefin having from 4 to 8 carbon atoms. Such copolymers and their preparation are well-known, and generally the isoolefin is a compound such as isobutylene and the diolefin is a compound such as butadiene or isoprene. Halogenated butyl rubbers are also well-known : chlorinated and brominated butyl rubber generally contains from 1.0 to 3.0 weight per cent bromine and from 0.05 to 0.5 weight per cent chlorine, respectively.

The elastomer film blend preferably comprises from 10 to 50 wt %, more preferably from 15 to 45 wt % and most preferably from 20 to 35 wt % of the olefinic elastomer based on the combined weight of thermoplastic and elastomer components. As mentioned above, such elastomeric component preferably is EPM or EPDM, but may for example be selected from the group consisting of EPM, EPDM, PIB, butyl rubber, halogenated butyl rubber, SBS, and mixtures and derivatives thereof. Alternatively stated, the garmant of the invention preferably comprises an outer layer, the blend of which comprises component (i) and (ii) in a weight ratio ((i):(ii)) of from 10:90 to 50:50, more preferably from 15:85 to 45:55 and most preferably from 20:80 to 35:65.

The film blend advantageously also comprises a normally liquid hydrocarbon process oil, preferably in an amount of 2-20 wt %, more preferably 4-15 wt % and most preferably 5-10 wt % based on the combined weight of thermoplastics, elastomer and oil components. Hydrocarbon oils which are particularly useful in the elastomer film component of the present invention function as process aids whose activity is uniquely enhanced in the presence of for example ethylene vinyl acetate copolymers, as plasticizers producing low modulus and enhanced elasticity in the solid state. Those especially useful are the normally liquid hydrocarbon processing and extender oils (ASTM D 2226) categorized as aromatic, naphthenic and paraffinic process oils of a medium viscosity range. Oils sold under the trademarks "Flexon", "Sunpar", "Marcol" and "Primol" have been found most especially useful.

In one preferred aspect the outer layer comprises a film formed from an elstomeric polymer blend composition comprising (based on the total weight of the composition) :

(a) 20 to 50 weight % of an EPM or EPDM elastomer ;

(b) 50 to 80 weight % of ethylene-vinyl acetate copolymer resin containing 9 to 40% by weight vinyl acetate ; and

(c) 2 to 20 weight % more preferably 5 to 10 weight % of normally liquid hydrocarbon process oil, the oil preferably being an aromatic, highly aromatic, naphthenic or paraffinic process extender oil.

Said blend composition preferably contains at least 4.5% by weight of vinyl acetate based upon the weight of the total composition and the blend composition preferably has a melt index at 190° C of 0.5 to 15.0 g/10 min.

In a further embodiment of the present invention the film blend comprises the aforesaid (a), (b) and (c) ingredients, together with (d) 0 to 45, more preferably 0 to 30, weight % of calcium carbonate as a filler and opacifying agent and (e) 0 to 2 weight % of a film processing slip agent or abherent based upon the weight of the total blend composition.

In yet another preferred aspect the outer layer film blend comprises from 10 to 40 wt % of an olefinic elastomer, not more than 12 wt % eg 2-12 wt % (more preferably not more than 10 wt %) of a normally liquid processing oil, and from 50 to 80 wt % of a thermoplastic ethylene (co)polymer, preferably EVA, the weights being based on the weight of the three component composition.

Especially preferred elastomeric films are those containing EPM or EPDM elastomers having at least 60 weight per cent ethylene and a molecular weight sufficient to provide a Mooney viscosity of from 20 to 60 ASTM D 1646 (ML 1 + 8 at 127° C). preferred ethylene-vinyl acetate copolymers are those containing 14% to 28% by weight vinyl acetate. Aromatic, naphthenic or paraffinic hydrocarbon plasticizer oils may be used. Paraffinic and naphthenic grades are commonly lighter in colour and lower in odour and therefore preferable. Aromatic oils are generally more compatible with other components and exhibit less surface migration when used at high percentages.

Calcium carbonate is an optional material for use in the preferred elastomeric film blend composition employed in the present invention and functions chiefly as a filler to reduce component cost. It may be used in fairly substantial amounts, up to 45, more preferably up to 30 parts by weight per 100 parts by weights of thermoplastics, elastomer and oil (if any).

It has been found useful in reducing film blocking, and it will impart an off-white cloudy appearance to the film. Calcium carbonate will also reduce tackiness in the finished film product.

Film processing slip agents or abherents are optional but preferable components of the film blend compositions used in the present invention. These materials are well-known in the art and are commonly employed in film manufacture as processing aids. Numerous materials are suitable but stearic acid and stearic acid derivatives such as calcium or zinc stearate or stearamide are particularly preferred. Other suitable abherents include the $C_{12}$-$C_{22}$ fatty acids and fatty acid amides and metal soaps, such as erucamid, silicones and natural and manufactured waxes such as glyceryl and glycol stearates, as well as inorganic abherent materials.

Most preferred proportions for preparing the film blend components of the present invention are (a) 25-50 parts by weight of olefinic elastomer, preferably EPM or EPDM ; (b) 40-55 parts by weight of thermoplastic ethylene homo- and/or co-polymer, preferably ethylene-vinyl acetate copolymer ; (c) 5-25 parts by weight of hydrocarbon oil and optionally 0.1 to 1.0% by weight of abherent, such as stearic acid or a fatty acid amide.

A preferred film composition containing calcium carbonate filler comprises:   (a) 20 to 45 parts by weight of elastomer such as an EPM or EPDM elastomer preferably having an ethylene content of from 45 to 80 weight per cent ;

(b) 35 to 50 parts by weight of a thermoplastic ethylene homo-and/or co-polymer such as ethylene-vinyl acetate copolymer preferably having a vinyl acetate content of 9 to 40 % by weight ;

(c) 5 to 20 parts by weight of a normally liquid hydrocarbon process oil, the oil being an aromatic, naphthenic or paraffinic extender oil ;

(d) 2 to 15 parts by weight of filler such as calcium carbonate ; and

(e) 0 to 1 % by weight, based on the total composition, of a film processing abherent,

the blend composition preferably having a melt index at 190° C (2.16 kg load) or 0.5 to 15 g/10 min. and in the case where the thermoplastic copolymer is EVA the blend composition preferably containing at least 4.5% by weight vinyl acetate based upon the total weight of the blend composition.

Garments of the invention having an outer layer, that is substantially all of the outer surface of the garment which is exposed when worn by the user, which is of the defined elastomeric nature may have the following advantages versus conventional films or tissues used as outer layers.

- Substantial elimination of noise (high degree of silence)

- Reduction of the cold plastic-like feeling

- Reduction in leakage problems currently requiring elastomeric strips and bands

- Higher degree of comfort resulting from the elastic nature of the outer layer

- Less movement of the product as a result of controlled surface friction of the outer layer.

With regard to the need to reduce leakage problems, it is a further important characteristic of garments of the present invention, and one which gives improved properties over similar garments known to the applicants, that the elastomeric outer film is generally orientated but has areas of reduced orientation providing extra grip in use.

It is known to use thermoplastic elastomeric film, dimensionally stabilized in a stretched condition (wherein stresses and strains in the film have been frozen in) for various applications. For example, the stretched thermoplastic elastomer may be applied to a substrate and later heated causing the thermoplastic elastomer to shrink and retain substantial elastic properties. One such use is disclosed in EP-A-0119815 wherein the stretched dimensionally stable thermoplastic elastomer is placed on a diaper waistband and reheated causing the thermoplastic elastomer to contract and revert to a heat stable elastic state. The diaper is thus provided with a flexible and stretchable waistband. A similar application of thermoplastic elastomers is disclosed in EP-A-0119827. These uses are distinguished form the present invention however in that the elastomeric film is applied to the existing outer layer which is not itself elastomeric, whereas in the present case, the whole of the outer layer is elastomeric and oriented with selected areas de-oriented to yield integrally improved elasticity and hence grip. The prior art methods suffer from the need to apply the elastomeric band in a separate stage, which may require adhesive techniques, and of course from the fact that the outer layer is if a conventional type which has all the disadvantages which are well known and which may be obviated by use of the elastomeric film outer layer employed in accordance with the present invention.

The preparation of oriented elastomeric film, and the heat shrink properties thereof are discussed in EP-A-0201331, the whole of the contents of which are incorporated herein by reference, although it is to be understood that the orientation and related techniques as discussed therein with reference to the particular film compositions defined therein are to be applied herein to the elastomeric films defined in Claim 1 hereof.

With regard to the method of the present invention, the techniques preferably employed in step (3) ie localised heat shrinkage are best described in relation to the techniques used in film manufacture to achieve appropriate orientation thereof. It will be appreciated, though, that film production and orientation does not of itself constitute one of the steps of the defined method. The following terms used herein have the meaning indicated:

"Draw ratio" - the ratio of the final stabilized length (after orientation and "snapback") of an oriented film and the initial length of the film before orientation. Draw ratio in unidirectional orientation is also equal to the ratio of the thickness of the stabilized oriented film and the initial unoriented film.

"Shrink force" - the force required to prevent shrinkage of an oriented film by application of heat.

"Shrink stress" - the shrink force per unit area (g/cm$^2$).

"Annealing" - a heat treatment process for reducing strains and stresses set up in the film during orientation. The process comprises maintaining the film while in stretched condition at the annealing temperature, for a period of time, followed by cooling the film to room temperature.

"Thermoplastic elastomer" - frequently called rubbery thermoplastics, are blends of a thermoplastic material and elastomer that are processable as a melt, at elevated temperatures, but exhibit properties similar to vulcanized elastomers at room temperature.

"Melt Index" (MI) - g/10 min (ASTM-D 1238; condition E)

A preferred method for preparing the thermoplastic elastomer film to be used as the outer layer in accordance with the present invention comprises stretching the film at a temperature below the melting point of the thermoplastic, preferably EVA, component at a draw ratio of from 1.3:1 to 6:1, annealing the stretched film, and cooling of the film to ambient temperature.

Preparation of compositions usable as elastomeric films in this invention can be achieved in several different ways. The various components may be brought into intimate contact by, for example, dry blending these materials and then passing the overall composition through a compounding extruder.

Alternatively, the components may be fed directly to a mixing device such as a compounding extruder, higher shear continuous mixer, two roll mill or an internal mixer such as a Banbury mixer. Optional ingredients such as those previously described can be added to the composition during this mixing operation. Overall, the objective is to obtain a uniform dispersion of all ingredients and this is readily achieved by inducing sufficient shear and heat to cause the plastics component(s) to melt. However, time and temperature of mixing should be controlled as is normally done by one skilled in the art so as to avoid molecular weight degradation.

Film from the resin compound may be manufactured by conventional tubular extrusion, (blown bubble process) or by cast extrusion, with the latter being preferred. In the cast extrusion process, the molten resin is extruded from an elongate die to the form of a web. The web is cast onto a chill roller, which solidifies the polymer, and finally would into a roll.

The extrusion temperatures, die temperatures, and chill roll temperatures will depend on the composition employed, by generally will be in the following ranges for typical elastomeric film compositions used in the present invention prepared by cast extrusion:

Melt Temperature      (°F) 350-450      (°C) 176-233
Die Temperature      (°F) 350-450      (°C) 176-233
Chill Roll Temperature      (°F) 70-130      (°C) 21-55

The process described above may also include a set of embossing rolls to chill and form the film.

In practice, the elastomer film may be produced using an in-line operation wherein the extruder and orientation system (eg tenter) are arranged in tandem to form the film by casting or melt embossing followed by orientation. Alternatively, these operations may be carried out separately.

Orientation of the film may be carried out in the machine direction (MD) or the transverse direction (TD) or both directions (biaxially) using conventional equipment and processes.

By way of example for orientation in the MD, the elastomeric film at an elevated temperature (but below the crystalline melting point of the thermoplastic component) is passed from a feed roll of film around two rollers driven at different surface speeds and finally to a takeup roller. The driven roller closest to the takeup roll is driven faster than the driven roller closest to the feed roll, such that the film is stretched between the driven rollers. The assembly may include a roller intermediate the second roller and takeup roller to cool the film. the second roller and the takeup roller may be driven at the same peripheral speeds to maintain the film in the stretched condition. If supplementary cooling is not used, the film will cool to ambient temperature on the take up roll.

The degree of stretch will depend upon the relative peripheral speeds of the driven rollers and the distance between the rollers. Stretch rates of 50 to 500 per cent/minute have been found to be satisfactory for most MD orientation applications.

To impart TD orientation to the film, film orientation is preferably carried out in a tentering device. The film is cast eg as described above or is unwound from a film roll and then gripped by the eges for processing through the orientation steps. The film is passed successively through a preheat step, a stretching step at elevated temperatures (eg from 37.7°C to a temperature slightly below the crystalline melting point of the thermoplastic ethylene homopolymer and/or copolymer component), an annealing step, and finally a cooling step. (Although cooling may be considered part of the annealing step, for convenience it is described as a separate step herein.) The preheat, orientation, and a portion of the annealing temperature is controlled at an elevated temperature but below the crystalline melting point of the thermoplastic polymer. Although, not essential, it is preferred that tension be maintained on the film during the annealing and cooling steps to minimize shrinkback. Upon cooling to ambient temperature (ie room temperature) or near ambient, the holding force may be released. The film may contract somewhat (snapback) in the TD but will retain a substantial portion if its stretched length.

The tenter operating conditions can vary within relatively wide ranges and will depend on the several variables including film compositions, film thickness, degree of orientation desired, annealing conditions, etc. The following is exemplary of a process for stretching 100 micron thick film (containing EVA) from 61 cm wide to a final width of about 152 cm, using a tenter manufactured by Marshall and Williams Company of Providence, Rhode Island.

## ESTIMATED FILM RANGE

| Step | Broad | Preferred | Typical | Approximate Time (Sec) |
|------|-------|-----------|---------|------------------------|
| Preheat | 100-160°F 37.7-71°C | 115-140°F 46-60°C | 125°F 51.6°C | 3.0 |
| Stretching | 100-160°F 37.7-71°C | 115-140°F 46-60°C | 125°F 51.6°C | 9.0 |
| Annealing | 100-160°F 37.7-71°C | 110-150°F 43-66°C | 120°F 48.8°C | 3.0 |
| Cooling | Ambient | Ambient | Ambient | 6.0 |

As indicated earlier, it is highly desirable to employ an annealing step in the process, preferably at about the same temperature as for orientation. Too high an annealing temperature gives loss of shrink force in the film. Annealing partially relieves the internal stress in the stretched film and dimensionally stabilizes the film for storage. It has been found that by annealing the film at a temperature of ± 22.2°C, preferably ± 11.1°C of the orientation temperature (but slightly below the crystalline melting point of the thermoplastic film component), undesirable shrinkage during storage is eliminated.

The preferred annealing temperature is between 43 and 54° C. Temperatures which result in excessive stress relieving should be avoided, since substantially frozen stresses and strains should remain after the process is completed.

Annealing can be accomplished by maintaining the film in the stretched condition at the annealing temperature. Preferably, however, the annealing and cooling is carried out while permitting the film to contract slightly, but still under stress. The guide rails of the tenter can be arranged in a converging manner to provide the annealing and cooling while the film contracts. The controlled shrinkback of from 5 to 30%, preferably between 15 to 25%, of the maximum stretched width has given particularly good results in eliminating storage shrinkage. This annealing and preshrinking removes some of the film stresses and strains so that shrinkage will not occur at storage temperature. However, the annealing and cooling does not remove all the frozen in stress and strain, since upon heating to elevated temperatures above storage temperature the film will shrink.

The degree of stretching may vary within wide ranges. A total draw ratio of 1.3:1 to 9:1 is preferred, and in the case of biaxially oriented film the undirectional draw ratios are preferably controlled to keep the total within this preferred range. Draw ratios of 1.3:1 to 6:1 are possible with 2:1 to 4:1 being especially preferred for TD tentering and MD orientation. The actual stretching will occur at higher ratios (1:5 to 9:1) to allow for controlled shrinkage and snapback.

The thermoplastic component of the film normally will determine the operating temperatures of the tentering and annealing operations. These operations are preferably carried out at temperatures not less than about 100° F (37° C) and below (preferably not more than about 10° C below) the crystalline melting point of the ethylene copolymer component. The annealing step preferably is at ± 20° F (11.1° C) of the orienting temperature. The crystalline melting point of EVA ranges from approximately 40° C to 94° C, depending on the VA content and MI, with the preferred EVAs having crystalline melting points between about 54° C and 71° C. For economics, orienting temperatures of 71° C and below are preferred.

In performing the method of the invention the outer layer elastomeric film which is provided may have been produced by the techniques described hereinbefore. The liquid permeable sheet constitutes the body contacting layer of the garment and may be of any suitable material having liquid pervious character and which is preferably of fine and soft texture to provide a reasonable measure of comfort to the wearer. Such layer could be a woven or a non-woven fabric and it could be of cotton, rayon, paper, synthetic materials, etc and could be of plural ply structure.

Intermediate the body contacting and outer layer in the assembled composite there is provided an absorbent layer, preferably of fluffed pulp material such as a core of fibrous cellulosic or other material.

In performing the method of the invention, step (2) is carried out by assembling the components in a manner which may conform with the techniques routinely employed for manufacturing such garments eg diapers. For example the assembly may be carried out on automatic lines by a lamination technique, with the various layers of the composite adhered eg with a hot-melt or other suitable adhesive. Typically a preferred disposable diaper incorporating the special outer layer as defined would be assembled by providing an absorbent layer which may be folded to provide a double thickness of absorbent in the diaper "pad" region. Prior to folding, a gel-forming material may be incorporated. The layer of absorbent material is incorporated into the diaper between the liquid impermeable outer layer and the liquid permeable liner sheet, and the assembly fixed by use of a standard commercially recommended adhesive for line, spot and elastic application eg hot-melt adhesive. The liner may be for example a non-woven material such as spunbonded polypropylene, a carded web, or a split or fibrillated film.

Step (3) of the method is carried out by applying heat to at least one selected local area of the oriented outer layer elastomeric film. The number, shape and location of the area(s) to which heat is applied depend on the nature of the garment and the type or orientation but for example in the case of a diaper the areas would be selected so as to correspond with a leg-gripping area and/or a waist-gripping area in use of the finished product. The provision of such grip regions in a finished diaper of course means that in use the diaper has improved fluid retention since contact between eg the leg and the leg portion of the diaper is improved by the increased elasticity in the region which has been subjected to heat treatment. The position of the film to which heat can advantageously be applied in accordance with the invention depends on the type of orientation in the elastomeric film. Thus if it is required only to have heat shrinkage in one direction of the film, then a mono-oriented film will suffice. However if areas of improved elasticity are required at angles on the film surface, then the film will have to be biaxially orientated.

In a preferred embodiment the heat is applied to produce a garment with a local area of reduced orientation at a portion of the elastomeric film which is of greater thickness than the remainder of the film. Such area of greater thickness enables a disproportionate increase in elasticity to be induced by reducing the orientation, hence leading to even more improved grip in the final garment.

The area of greater thickness may be produced during the course of film blowing or extrusion by adjustment of the die characteristics. Alternatively the increase in thickness may be generated by providing one or more folds in the elastomeric film. This technique is particularly useful when the area to be heated is at the film edge eg at leg or waistband of a diaper, when it is more convenient to introduce a fold via the film feed equipment. The fold may be continuous, or may be provided as a plurality of discontinuous folds which after extremely localised heat treatment at each fold effectively form a crimped region of the garment. The heating temperature in the extremely localised crimp regions will effect the crimping by melt-pressing, so inducing an improved elasticity in the crimp line by a size reduction ie mechanical technique. This will then be supplemented by the less localised heat treatment in accordance with step (3) of the method of the invention, along the line of crimps, which induces even more elasticity in the area by reducing the film orientation.

In the case of a diaper for example, it is convenient to use an elastomeric film which has been biaxially oriented and to heat treat such as to give both leg band and waistband grip regions. Alternatively a monoriented film may be used with heat treatment according to step (3) of the defined method being applied to local areas which constitute a leg band.

Improved waist band grip may then be provided by the plurality of folds/hot press technique described above, and in this case the localised higher temperatures used to hot-melt the crimps into place will not be detrimental to the orientation of the film in that region.

Heat treatment in accordance with step (3) of the method of the invention may be carried out by any convenient method, for example hot air jet or infra red heater, or even using laser heating or induction heating by high frequency electrical field.

The temperature of the localised heat treatment is preferably in about the same temperature range as has been hereinbefore described for the orientation process, that is at elevated temperatures eg. above 100° F (37.7° C) but below (although close to) the crystalline melting point of the thermoplastic component of the elastomeric film outer layer of the garment. The purpose of the heat treatment is to generate shrinkage of the stretch oriented film in the heated areas consistent with a good shrink force. There is a balance between shrink force and applied heat, since heating at a temperature which is too close to the crystalline melting point of the thermoplastic component of the film will lead to poorer heat shrinking. Shrinkage generally commences at a few degrees above storage temperature (ambient), reaching maximum at some temperature above the orientation temperature.

The optimum conditions are those which give shrinkage with a high shrink force, such that the contraction of the film in the heated local area also generates contraction in the composite garment of which the film comprises the outer layer. In this way a much improved grip is achieved in the heated regions during wearing of the garment eg diaper. The thermoplastic elastomer film preferably has a relatively high shrink force since the remainder of the composite of which it is a part resists shrinkage. The shrink force is determined by measuring the shrinkage of the film sample against an applied force and is referred to herein as weighted shrink. The weighted shrink properties differ markedly from free shrinkage (no applied weight) and hence is an important property in determining the suitability of a heat shrinkable film comprising the outer layer of a composite.

The elastomeric film of garments according to the invention preferably has a thickness of from 20-50 microns, more preferably 30-35 microns. Such thickness is especially suitable for the case where the garment is a diaper.

The following example illustrates the invention.

Example

A composition of the following formulation was mixed in a Banbury internal mixer by a double pass masterbatch/final mixing procedure. Thus the EPDM and antioxidant were ground for 30s; the EVA was added and mixed to 150°C; the ram was raised and the batch rolled for 20s; than the masterbatch was dumped. The masterbatch was then recharged to the mixer and the oil was added, followed by mixing to a full flux. This mixture was rolled for 20s with the ram raised, then mixed for 30s with ram dropped, then dumped. The composition was converted to film by cast extrusion at temperature 290°C; output 220 kg/hour; velocity 30m/minute :

|                     | wt %  |
|---------------------|-------|
| EPDM elastomer (1)  | 22.6  |
| EVA (2)             | 71.7  |
| Process oil (3)     | 5.6   |

500-1000 ppm of antioxidant (Irganox 1076 (trademark) of CIBA GEIGY) were also added.

The composition melt index was 3.25.

(1) Vistalon 3708 of Exxon Chemical Company, having 65 % ethylene content, medium diene content and Mooney viscosity (127°C) of 50.

(2) 28 % VA; melt index = 2 LD-767 of Exxon Chemical Company.

(3) Arco Prime 350 of Arco Chemical Company.

The film was then monoaxially drawn in TD on a Marshall and Williams tenter to a draw ratio of 2.42 at an orientation temperature of 52°C, annealed for 3 seconds at 50°C, and cooled to room temperature.

Experiments were conducted on the film to demonstrate shrinkage as a function of restraining force and shrinkage temperature. The film was processed as follows:

Initial Length    57.15 cm (22.5 inches)
Stretch Length    168 cm (66 inches)
Controlled Shrinkback Length    152 cm (60 inches)
Final Stabilized Length    127 cm (50 inches)
Film Gauge (Initial)    102 microns (avg)
Film Gauge (Final)    42 microns (avg)
Draw Ratio (Initial film gauge/Final film gauge)    2.42

Strips (three for each test) of the film were taken and subjected to shrinkage in an oven at a controlled temperature (49° C or 65.5° C) and at the following restraining forces: 0, 12g, 24g, 48g and 60g. Each strip was permitted to shrink for three minutes. The three-strip average for each test is given below:

Strip Length after Shrinkage (cm)*

| Film | Restraining Force (grams) | | | | | | Shrink Force** (grams) | Area (cm²) | Shrink Stress g/cm² |
|---|---|---|---|---|---|---|---|---|---|
| | 0 | 12 | 24 | 36 | 48 | 60 | | | |
| 49°C | 8.08 | 8.33 | 8.62 | 8.89 | 9.08 | 9.33 | 91 | .010 | 9100 |
| 65.6°C | 5.83 | 6.26 | 6.98 | 7.73 | 8.38 | 9.43 | 73 | .011 | 6636 |

* three strip sample average

** calculated

These data demonstrate that for this film the shrinkage is generally linear with respect to the applied force. This permits calculating the shrink force and shrink stress. Note that the shrink temperature of 65.5° C is higher than the orientation temperature 49° C is lower than that temperature. The higher temperature results in more shrinkage since more stresses are relieved.

The film not used for shrink testing was cut to shape and used as the outer layer of a composite which, on assembly using a commercially available adhesive, constituted a diaper having the film as outer layer, a liquid permeable liner and a liquid absorbent layer disposed therebetween. Assembly was by hand, with the liner and absorbent layer being taken from a commercially available diaper (Procter and Gamble Company, Pampers brand). Of course in commercial production assembly is a rapid line automatic process.

The diaper was then subjected to localised heat treatment using a hot-air jet directed to the leg regions of the outer layer. The air temperature and time of application to the outer layer was judged sufficient to allow the elastomeric film to reach a temperature above the orientation temperature but below the crystalline melting point of the film. This resulted in loss of orientation and hence shrinkage and increased elasticity in the heated region.

The diaper made in the Example was judged to be more comfortable and less noisy than those using non-elastomeric outer layers; moreover the diaper which had been heat treated as described was judged to provide an improved fit and better grip (and hence better liquid retention in use) both than conventional diapers having non-elastomeric outer layers (but optionally applied strip of elastomeric material) and also than the diaper before it had been subjected to the localised heat treatment step.

Diaper produced in a fashion corresponding with that described herein before, but in which the outer layer blend comprises 30 wt % EPDM, 65 wt % EVA and 5 % process oil, plus 10 % calcium carbonate (based on the total of EPDM, EVA and oil) are judged also to have the above-mentioned advantageous properties making then particularly suitable as consumer products.

## Claims

1. A disposable liquid absorbent garment having at least one elastic grip region for improved body fit in use, which garment comprises in combination :
(a) a liquid impervious outer layer;
(b) a liquid permeable liner;
(c) an absorbent layer disposed between the outer layer and the liner
characterised in that the outer layer comprises an elastomeric film comprising a blend of (i) an olefinic elastomer and (ii) a thermoplastic ethylene homopolymer and/or copolymer and in that the elastomeric film is oriented save for at least one local area of reduced orientation and increased elasticity which forms the elastic grip region.

2. A garment according to claim 1 which comprises a diaper.

3. A garment according to claim 2 wherein the elastic grip regions of the diaper correspond to those areas which are adapted to fit the legs and/or waist of the wearer in use.

4. A garment according to claim 1, 2 or 3 in which the blend comprises (i) and (ii) in a weight ratio of from 10:90 to 50:50.

5. A garment according to claim 4 wherein the weight ratio of (i):(ii) is in the range 15:85 to 45:55, more preferably 20:80 to 35:65.

6. A garment according to any of the preceding claims in which the ethylene copolymer (ii) comprises an ethylene/vinyl acetate and/or ethylene/acrylic acid and/or ethylene/methacrylic acid and/or ethylene/methyl acrylate and/or ethylene/butyl acrylate copolymer.

7. A garment according to any of the preceding claims in which the olefinic elastomer component (i) comprises an ethylene-propylene elastomer (EPM) and/or an ethylene/propylene-diene elastomer (EPDM).

8. A garment according to claim 7 wherein the EPM or EPDM has an ethylene content of from 45-90 wt.% and a Mooney viscosity (ML 1 + 8 at 127° C) of from 15-80.

9. A garment according to any of the preceding claims in which the blend comprises from 2-20 wt % of normally liquid hydrocarbon oil, based on the total weight of (i) + (ii) + oil.

10. A garment according to claim 9 wherein the blend comprises from 4-15 wt % oil, more preferably from 5-10 wt % oil.

11. A garment according to any of the preceding claims in which the outer layer comprises a film formed from a thermoplastic elastomeric polymer blend composition comprising :
(a) 20-50 parts by wt. of an EMP or EPDM elastomer;
(b) 50-80 parts by wt. of an ethylene/vinyl acetate copolymer containing from 9-40 % by wt. of vinyl acetate; and
(c) 2-20 parts by wt. of normally liquid hydrocarbon oil.

12. A garment according to claim 11 in which the blend further comprises (d) from 0-25 % by wt. of calcium carbonate as a filler and opacifying agent and (e) from 0-2 % by wt. of a film processing slip agent or abherent based upon the weight of the total blend composition.

13. A garment according to any one of the preceding claims wherein the elastomeric film is biaxially oriented.

14. A garment according to any of claims 1-12 wherein the elastomeric film is mono-oriented.

15.. A garment according to any of the preceding claims wherein the local area of reduced orientation is at a portion of the film which is of greater thickness than the remainder of the film.

16. A garment according to claim 15 wherein the local area of reduced orientation is an edge region of the garment and the portion of greater thickness constitutes a fold in the film.

17. A garment according to claim 16 wherein the fold is continuous along the edge region.

18. A garment according to claim 16 wherein the edge region has a plurality of discontinuous folds comprising crimps along the edge region.

19. A method of producing a disposable liquid absorbent garment having at least one elastic grip region for improved body fit in use, which comprises

(1) providing (i) an elastomeric film comprising a blend of a thermoplastic ethylene homopolymer and/or copolymer and an olefinic elastomer, and film being oriented and being dimensionally stable, thermally unstable in the oriented condition and contractable to a thermally stable and more elastic condition by application of heat; (ii) a liquid permeable sheet; and (iii) an absorbent material;

(2) assembling the film, sheet and absorbent material to form a shaped composite which comprises a liquid impervious outer layer comprising the elastomeric film, a liquid permeable liner comprising the sheet, and an absorbent layer comprising the absorbent material disposed between the outer layer and the liner; and

(3) applying heat to at least one selected local area of the outer layer to reduce the film orientation in that area and thereby contract the film in that area to a thermally stable and more elastic condition to constitute the elastic grip region.

20. A method according to claim 19 wherein the elastomeric film has the characterising features as defined in any one of claims 4-18.

21. A method according to claim 19 or 20 wherein the film which is provided has been stretch oriented in a total draw ratio of from 1.3:1 to 9:1.

22. A method according to claim 19, 20 or 21 wherein the film is oriented at an elevated temperature below the crystalline melting point of the thermoplastic component of the blend.

23. A method according to claim 22 wherein after orientation the film is annealed at a temperature within 22.2 degrees C of the orientation temperature but below the crystalline melting point of the thermoplastic component of the blend.

24. A method according to any of claims 19-23 wherein step (3) is performed at a temperature of no more than 10 degrees C below the crystalline melting point of the thermoplastic component of the blend.

25. A method according to claims 19-24 wherein the elastomeric film is from 20-50 microns thick in the oriented condition.

26. A method according to claims 19-25 wherein the elastomeric film is provided with thickened regions which form the selected local areas to which local heat is supplied in step (3).

27. A method according to claims 19-26 wherein step (3) is performed on a continuous edge portion of the shaped composite.

28. A method according to any of claims 19-27 wherein step (3) is performed on a plurality of discontinuous folds along an edge portion of the shaped composite to generate an elastic grip region of reduced orientation which also comprises a plurality of crimps.

29. A method according to claims 19-28 wherein the shaped composite comprises a diaper.